# EUROPEAN PATENT SPECIFICATION

(11) **EP 2 903 687 B1**
(45) Date of publication and mention of the grant of the patent: **14.09.2016**
(21) Application number: 13779441.8
(22) Date of filing: 26.09.2013
(51) Int. Cl.: A61N 1/05, A61N 1/372, A61N 1/36, A61B 90/00, A61B 34/10

(54) **DIRECTIONAL LEADS**
DIREKTIONALE ELEKTRODEN
SONDES DIRECTIONNELLES

(30) Priority: 03.10.2012 US 201261744834 P
(43) Date of publication of application: 12.08.2015
(73) Proprietor: Boston Scientific Neuromodulation Corporation, Valencia, CA 91355 (US)
(72) Inventor: CARCIERI, Stephen, Los Angeles, California 90026 (US); RANU, Emarit, Fort Collins, Colorado 80525 (US); HAUT, Harold, Sea Cliff, New York 11579 (US)
(74) Representative: Vossius & Partner Patentanwälte Rechtsanwälte mbB
(86) International application number: PCT/US2013/062011
(87) International publication number: WO 2014/055328

(56) References cited:
- WO-A1-2004/084749
- WO-A1-2009/102508
- WO-A1-2011/092613
- WO-A2-2012/109338

## Description

### FIELD OF THE INVENTION

The present invention relates to tissue stimulation systems having radially segmented electrodes.

### BACKGROUND OF THE INVENTION

Deep brain stimulation (DBS) and other related procedures involving the implantation of leads and catheters in the brain are increasingly used to treat such conditions as Parkinson's disease, dystonia, essential tremor, seizure disorders, obesity, depression, motor control disorders, and other debilitating diseases. During these procedures, a catheter, lead, or other medical device is strategically placed at a target site in the brain. Locating the "best" or optimal site in the brain for deep brain stimulation can be a painstaking procedure.

Implantation of a lead for DBS generally involves preliminarily identifying a promising brain site by recording individual cell activity with a microelectrode. Microelectrode recording is generally performed with a microelectrode recording (MER) system, which includes a small diameter electrode with a relatively small surface area optimal for recording single cell activity. The microelectrode, which may essentially be an insulated wire that has at least the distal portion uninsulated to receive electrical signals, functions as a probe to locate an optimal site in the brain for deep brain stimulation. Activity detected through the microelectrode is then recorded by the MER system. The recorded output of a microelectrode advanced along a path through the brain is referred to as a recording tract.

A microelectrode passing through different areas of the brain produces an electrical signature unique to each area. For example, patterns of neuronal activity in the sub-thalamic nucleus (STN) are different from patterns of neuronal activity in the substantia nigra. Based on these unique patterns, microelectrode recording can be used to delineate the exact borders of the different brain structures (e.g., the STN) in order to help identify the most effective position for the permanent stimulation electrodes. In current techniques, as the microelectrode is advanced, the electrophysiologist marks the length of the STN as a colored line along an axis on a piece of paper (tick marks on the axis represent distance from the target). The length of the line is determined by concurrent microelectrode recording measurements. A sticker representing the DBS lead electrodes can then be placed over the colored line to determine the depth the lead should be inserted into the brain and what electrodes on the lead may be activated as part of therapeutic intervention. This method is effective for mapping one dimension (e.g., depth) of the STN and using that information to guide lead placement in that dimension.

While this technique is quite useful for non-directional DBS lead designs, in the cases where radially directional DBS lead designs, which may include radially segmented electrodes, are used, the mapping of more than one dimension may be required. In particular, for these lead designs, it would be desirable to know the radial orientation of the segmented electrodes, as well as their depth in the brain. There, thus, remains a need for a method of using MER data to guide DBS lead orientation as well as depth.

WO 2009/102508 A1 relates to an electrode device having directional electrodes for use in deep brain stimulation. It also discloses an electrode assembly comprising an elongate lead and a lead guide that are engageable with each other in a coaxial relationship, and when engaged, are rotationally fixed in relation to each other. It further discloses an elongate lead comprises a radiologically-visible feature for indicating the orientation of the elongate lead and also an electrode system that is capable of determining the position and/or orientation of an electrode positioned within a body.

### SUMMARY OF THE INVENTION

The scope of the present invention is defined by the claims.

In accordance with one aspect of the present disclosure, a method for implanting a lead within brain tissue of a patient is provided. The lead includes at least one set of radially segmented electrodes on a distal end of the lead. The method includes performing a plurality of microelectrode recordings through a respective plurality of recording tracts in the brain tissue; based on the microelectrode recordings, creating a three-dimensional map of a brain structure (e.g., a sub-thalamic nucleus) within the brain tissue; and positioning a graphical representation of the at least one set of radially segmented electrodes over the map of the brain structure to create a graphical depiction of a desired depth and a desired radial orientation of the lead within the brain tissue. The graphical representation of the at least one set of radially segmented electrodes may be a two dimensional graphical representation of the at least one set of radially segmented electrodes. The method further includes implanting the lead in the brain tissue in accordance with the desired depth and desired radial orientation.

The method may also include using a radially directional device to determine the actual radial orientation of the lead before implanting the lead. The radially directional device may include a radially directional ruler for determining the actual radial orientation of the lead based on electrical continuity between the electrodes in the set of radially segmented electrodes and contacts on a proximal end of the lead. The set of radially segmented electrodes may include at least two electrodes, and the radially directional ruler may include a distal contact. The method may further include coupling the lead to the radially directional ruler, such that one of the electrodes in the set of radially segmented electrodes is electrically coupled to the distal contact on the radially directional ruler, wherein the radially directional ruler comprises an indicator for indicating which one of the electrodes is in contact with the radially directional ruler.

The radially directional device may include a laser, and the actual radial orientation of the lead may be determined by using optical methods. For example, the lead may include a laser marker in alignment with one of the electrodes in the set of radially segmented electrodes, and the radially directional device may include an indicator for indicating when the laser marker is in alignment with the laser. In another example, the lead may include optically opaque fenestrations, and the radially directional device may include an emitter-detector. Determining the actual radial orientation of the lead may include rotating the lead about its longitudinal axis and using the emitter-detector to count the fenestrations.

The method may further include using a computer to create the map of the brain structure and position of the graphical representation of the at least one set of radially segmented electrodes. Positioning the graphical representation of the at least one set of radially segmented electrodes may include using a pointing device to drag and drop the graphical representation of the at least one set of radially segmented electrodes.

In accordance with another aspect of the present disclosure, a device for determining a radial orientation of a lead is provided. The lead includes at least one set of radially segmented electrodes on a distal end of the lead, and at least two contacts on a proximal end of the lead. Each electrode in the set of radially segmented electrodes is coupled to one of the contacts on the proximal end of the lead.

The device includes a radially directional ruler comprising a distal contact and at least two proximal contacts, and an indicator (e.g., a LED) for indicating which one of the electrodes in the set of radially segmented electrodes is in contact with the radially directional ruler. The radially directional ruler is configured for contacting the lead such that the ruler distal contact is coupled to one of the electrodes in the set of radially segmented electrodes, and the ruler proximal contacts are coupled to the lead proximal contacts, thereby forming a closed circuit between the one of the electrodes coupled to the ruler distal contact and the lead proximal contact that corresponds to the one of the electrodes. The indicator is configured for indicating which one of the lead proximal contacts is in the closed circuit.

The set of radially segmented electrodes may include three electrodes, and the radially directional ruler may include three proximal contacts. The radially directional ruler may include at least two open circuits, wherein each open circuit comprises the distal contact and one of the proximal contacts. The radially directional ruler may be configured for being attached to a stereotactic frame.

In accordance with yet another aspect of the present disclosure, an external control device for planning a depth and a radial orientation of a lead to be implanted within brain tissue of a patient is provided. The lead includes at least one set of radially segmented electrodes on a distal end of the lead. The external control device includes a user interface configured for receiving input from a microelectrode recording system, and for receiving input from a user, wherein the microelectrode recording system input comprises information related to a plurality of microelectrode recordings through a respective plurality of recording tracts in the brain tissue, and wherein the user input comprises information related to a desired position of the lead relative to the brain tissue. The external control device further includes control circuitry configured for, in response to the microelectrode recording input, creating a three-dimensional map of a brain structure within the brain tissue, and, in response to the user input, positioning a graphical representation of the at least one set of radially segmented electrodes over the map of the brain structure to create a graphical depiction of a desired depth and a desired radial orientation of the lead within the brain tissue. The graphical representation of the at least one set of radially segmented electrodes may be a two dimensional graphical representation of the at least one set of radially segmented electrodes.

The control circuitry may be configured for dragging and dropping the graphical representation of the at least one set of radially segmented electrodes over the map of the brain structure. The control circuitry may be configured for: selecting the graphical representation of the at least one set of radially segmented electrodes by coupling a pointing device to the graphical representation of the at least one set of radially segmented electrodes; dragging the graphical representation of the at least one set of radially segmented electrodes by moving the pointing device; and dropping the graphical representation of the at least one set of radially segmented electrodes by decoupling the pointing device from the dragged graphical representation of the at least one set of radially segmented electrodes.

The present invention is defined in the claims. Other and further aspects and features of the invention will be evident from reading the following detailed description of the preferred embodiments, which are intended to illustrate, not limit, the invention.

### BRIEF DESCRIPTION OF THE DRAWINGS

The invention will be described and explained with additional specificity and detail through the use of the accompanying drawings in which:
**Fig. 1** is a plan view of a Deep Brain Stimulation (DBS) system constructed in accordance with one embodiment of the present inventions;
**Fig. 2** is a profile view of an implantable pulse generator (IPG) and neurostimulation leads used in the DBS system of **Fig. 1**;
**Fig. 3** is a cross-sectional view of one of the neurostimulation leads of **Fig. 2**, taken along the **line 3-3**;
**Fig. 4** is a cross-sectional view of a patient's head showing the implantation of stimulation leads and an IPG of the DBS system of **Fig. 1**;
**Fig. 5** is a graphical representation of a map of a brain structure;
**Fig. 6** is a two dimensional unrolled representation of the electrode array on one of the neurostimulation leads of **Fig. 2**;
**Fig. 7** is a graphical representation of the electrode array of **Fig. 6** positioned over the map of the brain structure of **Fig. 5**;
**Fig. 8** is a block diagram of the components of an external control device for planning a depth and radial orientation of one of the neurostimulation leads of **Fig. 2** to be implanted within brain tissue;
**Fig. 9** is a plan view of a radially directional device being used with a neurostimulation lead of **Fig. 2****,** in accordance with one embodiment of the present invention;
**Fig. 10** is a plan view of a radially directional device being used with a neurostimulation lead of **Fig. 2****,** which falls not under the scope of the present invention; and
**Fig. 11** is a perspective view of a radially directional device in use with stereotactic equipment for introducing a neurostimulation lead into the brain of a patient.

### DETAILED DESCRIPTION OF THE EMBODIMENTS

The description that follows relates to a deep brain stimulation (DBS) system. However, it is to be understood that the while the invention lends itself well to applications in DBS, the invention, in its broadest aspects, may not be so limited. Rather, the invention may be used with any type of implantable electrical circuitry used to stimulate tissue. For example, the present invention may be used as part of a pacemaker, a defibrillator, a cochlear stimulator, a retinal stimulator, a stimulator configured to produce coordinated limb movement, a cortical stimulator, a spinal cord stimulator, peripheral nerve stimulator, microstimulator, or in any other neural stimulator configured to treat urinary incontinence, sleep apnea, shoulder subluxation, headache, etc.

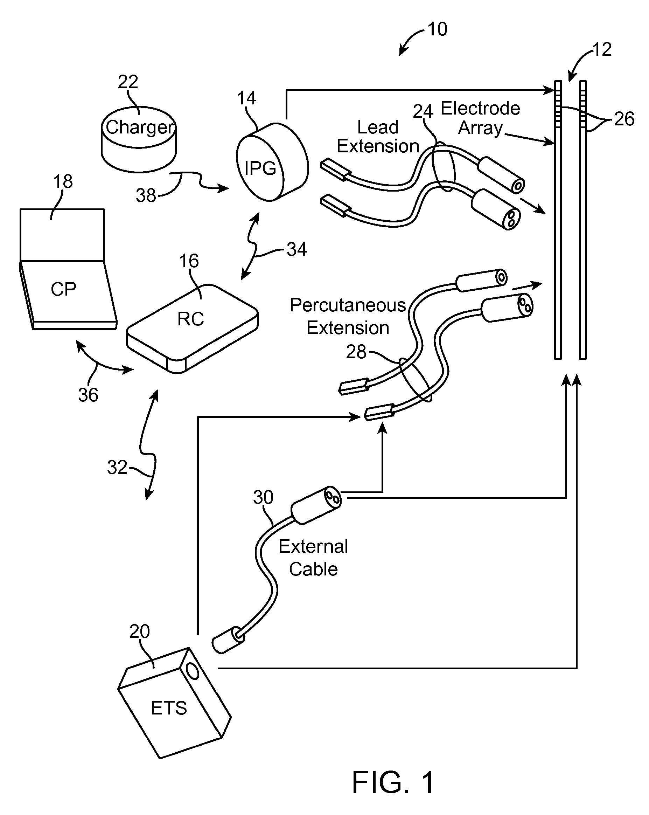

Turning first to **Fig. 1****,** an exemplary DBS neurostimulation system 10 generally includes at least one implantable stimulation lead 12 (in this case, two), a neurostimulator in the form of an implantable pulse generator (IPG) 14, an external remote controller RC 16, a clinician's programmer (CP) 18, an External Trial Stimulator (ETS) 20, and an external charger 22.

The IPG 14 is physically connected via one or more percutaneous lead extensions 24 to the neurostimulation leads 12, which carry a plurality of electrodes 26 arranged in an array. In the illustrated embodiment, the neurostimulation leads 12 are percutaneous leads, and to this end, the electrodes 26 may be arranged in-line along the neurostimulation leads 12. As will be described in further detail below, the IPG 14 includes pulse generation circuitry that delivers electrical stimulation energy in the form of a pulsed electrical waveform (i.e., a temporal series of electrical pulses) to the electrode array 26 in accordance with a set of stimulation parameters.

The ETS 20 may also be physically connected via percutaneous lead extensions 28 and an external cable 30 to the neurostimulation leads 12. The ETS 20, which has pulse generation circuitry similar to that of the IPG 14, also delivers electrical stimulation energy in the form of a pulsed electrical waveform to the electrode array 26 accordance with a set of stimulation parameters. The major difference between the ETS 20 and the IPG 14 is that the ETS 20 is a non-implantable device that is used on a trial basis after the neurostimulation leads 12 have been implanted and prior to implantation of the IPG 14, to test the responsiveness of the stimulation that is to be provided. Thus, any functions described herein with respect to the IPG 14 can likewise be performed with respect to the ETS 20.

The RC 16 may be used to telemetrically control the ETS 20 via a bidirectional RF communications link 32. Once the IPG 14 and stimulation leads 12 are implanted, the RC 16 may be used to telemetrically control the IPG 14 via a bidirectional RF communications link 34. Such control allows the IPG 14 to be turned on or off and to be programmed with different stimulation parameter sets. The IPG 14 may also be operated to modify the programmed stimulation parameters to actively control the characteristics of the electrical stimulation energy output by the IPG 14. As will be described in further detail below, the CP 18 provides clinician detailed stimulation parameters for programming the IPG 14 and ETS 20 in the operating room and in follow-up sessions.

The CP 18 may perform this function by indirectly communicating with the IPG 14 or ETS 20, through the RC 16, via an IR communications link 36. Alternatively, the CP 18 may directly communicate with the IPG 14 or ETS 20 via an RF communications link (not shown). The clinician detailed stimulation parameters provided by the CP 18 are also used to program the RC 16, so that the stimulation parameters can be subsequently modified by operation of the RC 16 in a stand-alone mode (i.e., without the assistance of the CP 18).

The external charger 22 is a portable device used to transcutaneously charge the IPG 14 via an inductive link 38. For purposes of brevity, the details of the external charger 22 will not be described herein. Details of exemplary embodiments of external chargers are disclosed in U.S. Patent No. 6,895,280. Once the IPG 14 has been programmed, and its power source has been charged by the external charger 22 or otherwise replenished, the IPG 14 may function as programmed without the RC 16 or CP 18 being present.

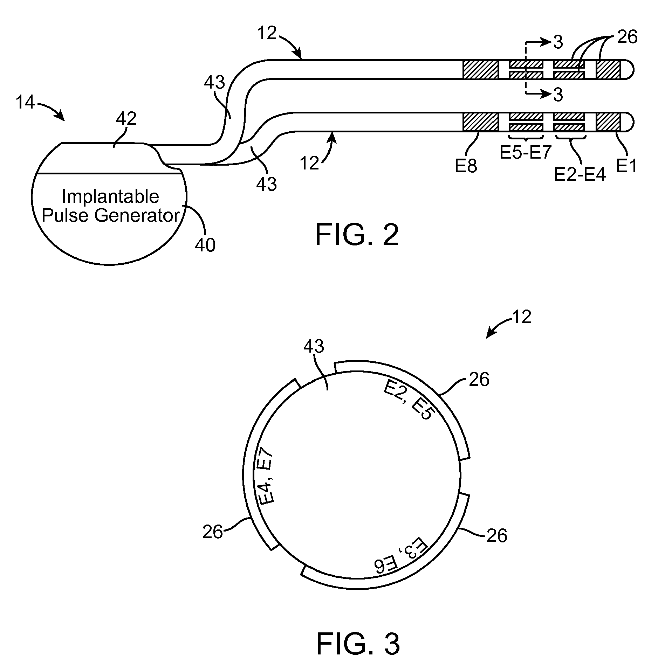

Referring to **Fig. 2****,** the IPG 14 comprises an outer case 40 for housing the electronic and other components (described in further detail below), and a connector 42 to which the proximal end of the neurostimulation lead 12 mates in a manner that electrically couples the electrodes 26 to the internal electronics (described in further detail below) within the outer case 40. The outer case 40 is composed of an electrically conductive, biocompatible material, such as titanium, and forms a hermetically sealed compartment wherein the internal electronics are protected from the body tissue and fluids. In some cases, the outer case 40 may serve as an electrode.

Each of the neurostimulation leads 12 comprises an elongated cylindrical lead body 43, and the electrodes 26 take the form of ring electrodes or segmented electrodes that are circumferentially and axially disposed about the lead body 43. By way of non-limiting example, and with further reference to **Fig. 3****,** one neurostimulation lead 12 may carry eight electrodes, arranged in a 1-3-3-1 configuration with two sets of radially segmented electrodes positioned between two ring electrodes. In this embodiment, each set of radially segmented electrodes includes three electrodes. The distal-most electrode is ring electrode E1. Proximal to ring electrode E1 is a first set of three radially segmented electrodes E2-E4. Proximal to the first set of radially segmented electrodes is a second set of three radially segmented electrodes E5-E7. The proximal-most electrode is ring electrode E8. It should be well understood that the arrangement of the electrodes 26 on the lead 12 may be in any desired configuration. The lead 12 carries at least one set of radially segmented electrodes, and may carry only radially segmented electrodes, or any combination of ring electrodes and radially segmented electrodes. Each set of radially segmented electrodes may include two, three, four, or more electrodes.

Further details describing the construction and method of manufacturing percutaneous stimulation leads are disclosed in U.S. Patent Nos. 8,019,439 and 7,650,184.

As will be described in further detail below, the IPG 14 includes a battery and pulse generation circuitry that delivers the electrical stimulation energy in the form of a pulsed electrical waveform to the electrode array 26 in accordance with a set of stimulation parameters programmed into the IPG 14. Such stimulation parameters may comprise electrode combinations, which define the electrodes that are activated as anodes (positive), cathodes (negative), and turned off (zero), percentage of stimulation energy assigned to each electrode (fractionalized electrode configurations), and electrical pulse parameters, which define the pulse amplitude (measured in milliamps or volts depending on whether the IPG 14 supplies constant current or constant voltage to the electrode array 26), pulse duration (measured in microseconds), pulse rate (measured in pulses per second), and burst rate (measured as the stimulation on duration X and stimulation off duration Y). The IPG 14 may be capable of delivering the stimulation energy to the array 26 over multiple channels or over only a single channel.

Electrical stimulation will occur between two (or more) activated electrodes, one of which may be the IPG case. Simulation energy may be transmitted to the tissue in a monopolar or multipolar (e.g., bipolar, tripolar, etc.) fashion. Monopolar stimulation occurs when a selected one of the lead electrodes 26 is activated along with the case of the IPG 14, so that stimulation energy is transmitted between the selected electrode 26 and case. Bipolar stimulation occurs when two of the lead electrodes 26 are activated as anode and cathode, so that stimulation energy is transmitted between the selected electrodes 26. Multipolar stimulation occurs when at least three of the lead electrodes 26 are activated, e.g., two as anodes and the remaining one as a cathode, or two as cathodes and the remaining one as an anode.

In the illustrated embodiment, IPG 14 can individually control the magnitude of electrical current flowing through each of the electrodes 26. In this case, it is preferred to use current generators, wherein individual current-regulated amplitudes from independent current sources for each electrode may be selectively generated. Although this system is optimal to take advantage of the invention, other stimulators that may be used with the invention include stimulators having voltage regulated outputs. While individually programmable electrode amplitudes are optimal to achieve fine control, a single output source switched across electrodes may also be used, although with less fine control in programming. Mixed current and voltage regulated devices may also be used with the invention.

Further details discussing the detailed structure and function of IPGs are described more fully in U.S. Patent Nos. 6,516,227 and 6,993,384.

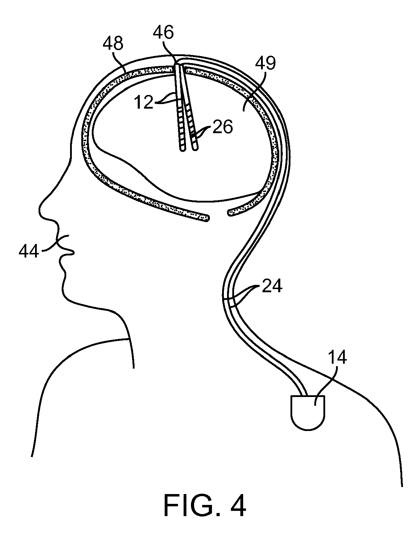

As shown in **Fig. 4****,** two percutaneous neurostimulation leads 12 are introduced through a burr hole 46 (or alternatively, two respective burr holes) formed in the cranium 48 of a patient 44, and introduced into the parenchyma of the brain 49 of the patient 44 in a conventional manner, such that the electrodes 26 are adjacent a target tissue region, the stimulation of which will treat the dysfunction (e.g., the ventrolateral thalamus, internal segment of globus pallidus, substantia nigra pars reticulate, subthalamic nucleus, or external segment of globus pallidus). Thus, stimulation energy can be conveyed from the electrodes 26 to the target tissue region to change the status of the dysfunction. Due to the lack of space near the location where the neurostimulation leads 12 exit the burr hole 46, the IPG 14 is generally implanted in a surgically-made pocket either in the chest, or in the abdomen. The IPG 14 may, of course, also be implanted in other locations of the patient's body. The lead extension(s) 24 facilitates locating the IPG 14 away from the exit point of the neurostimulation leads 12.

Prior to implanting the neurostimulation leads 12 into the patient's brain tissue, microelectrode recording (MER) is performed to determine optimal placement of the leads 12. Thus, a method for implanting a lead within brain tissue of a patient includes a step of performing a plurality of microelectrode recordings through a respective plurality of recording tracts in the brain tissue. An electrode guide tool, such as a Ben-Gun, having multiple (e.g., five) parallel channels may be used for guiding a microelectrode into the brain tissue in multiple different locations. While the microelectrode is being advanced through each recording tract, patterns of neuronal activity recorded by the microelectrode indicate which brain structures the microelectrode is passing through. Each brain structure has a unique pattern of neuronal activity. In this manner, MER may be used to determine the borders of a target brain structure. For example, if the target brain structure is the subthalamic nucleus (STN), the borders of the STN may be determined based on the section of the recording tract during which the pattern of neuronal activity unique to the STN is encountered.

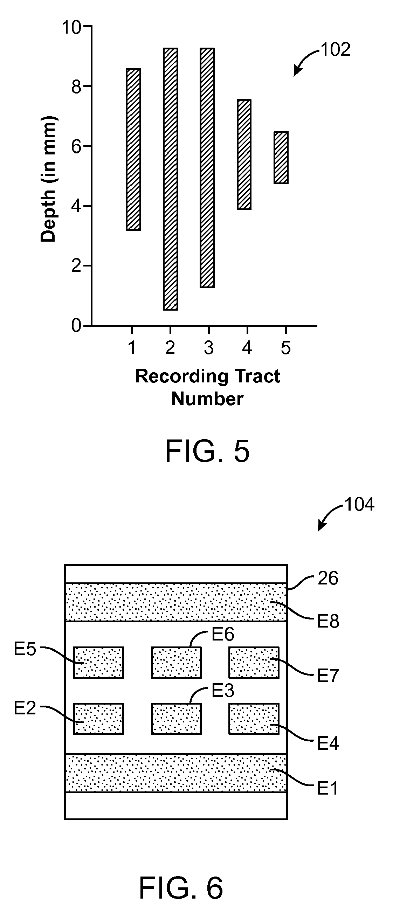

Based on the plurality of microelectrode recordings, a three-dimensional map of a brain structure (e.g., the STN) within the brain tissue is created. The map may be created on paper or may be created electronically. While the mapping may be done in three dimensions, the representation of the map of the brain structure may be easily visualized in two dimensions. For example, **Fig. 5** depicts a two-dimensional representation of a map 102 of the STN, where the portion of the STN that is accessed by passing through channel 1 of the electrode guide tool extends between about 3mm and about 8mm below the distal end of a guide cannula, the portion of the STN that is accessed by passing through channel 2 of the electrode guide tool extends between about 1 mm and about 9mm below the distal end of the guide cannula, etc. The recording tracts may be arranged in a cylindrical pattern, and the map 102 may be an "unrolled" or "unwrapped" representation of the cylinder about which the recording tracts are arranged. While the exemplary map 102 shown in **Fig. 5** may be a map of the STN, it should be well understood that other brain structures (e.g., the ventrolateral thalamus, internal segment of globus pallidus, substantia nigra pars reticulate, or external segment of globus pallidus) may be mapped using a substantially similar method to that described above.

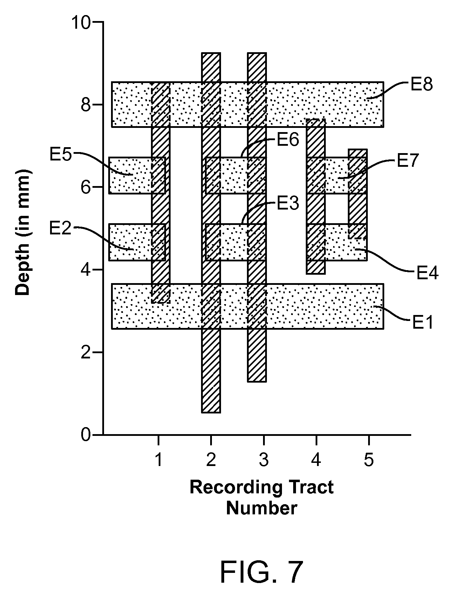

After the map 102 is created as shown in **Fig. 5****,** a graphical representation of at least one radially segmented electrode is positioned over the map 102 of the brain structure to create a graphical depiction of a desired depth and a desired radial orientation of the lead within the brain tissue. A graphical representation 104 of the electrodes 26 of the lead 12 is shown in **Fig. 6** as an "unrolled," or "unwrapped," two dimensional representation of the electrodes 26 that is to scale with the map 102 of the brain structure. The graphical representation 104 of the electrodes 26 can be positioned over the map 102 of the brain structure as shown in **Fig. 7****.** By positioning the graphical representation 104 of the electrodes 26 over the map 102, the desired depth of the lead 12 can be determined. In addition to the desired depth, the desired orientation of the radially segmented electrodes 26 can be determined. For example, as shown in **Fig. 7****,** the desired orientation of the lead 12 is with electrodes E2 and E5 facing towards the tissue of recording tract 1, electrodes E3 and E6 facing towards the tissue of recording tracts 2 and 3, and electrodes E4 and E7 facing towards the tissue of recording tracts 4 and 5. The method of generating and superimposing the map 102 of the brain structure and graphical representation 104 of the electrodes 26 may be performed either electronically or non-electronically.

If the method is being performed electronically, the graphical representation 104 of the electrodes 26 can be selected and dragged into the desired position on the map 102 using an electronic pointing device (e.g., a mouse cursor, or finger or stylus on a touch screen). The electrodes 26 can be re-ordered by swiping the electrodes so that the right-most electrode becomes the left-most electrode, or vice-versa, and the remaining electrodes follow in sequence. This swiping of the two dimensional representation 104 of the electrodes 26 represents rotating the lead 12.

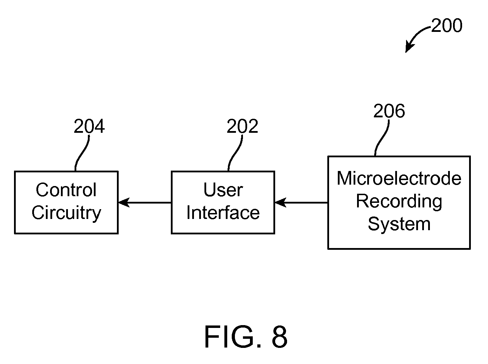

The method may be performed electronically using an external control device, such as that depicted in **Fig. 8****.** The external control device 200 includes a user interface 202 and control circuitry 204. The user interface 202 may include a user input device (e.g., a mouse, keyboard, touch screen, etc.) and a display. The user interface 202 receives input from a microelectrode recording system 206. Input from the microelectrode recording system 206 may include information related to the plurality of microelectrode recordings performed in the respective plurality of recording tracts in the brain tissue. In response to the input from the microelectrode recording system 206, the control circuitry 204 creates a three-dimensional map of the brain structure within the brain tissue. The three-dimensional map may be displayed on the user interface 202 as a two-dimensional representation, such as the map 102 depicted in **Fig. 5****.** The user interface 202 is further configured for receiving input from a user, such as information related to a desired position of the lead 12 relative to the brain tissue. In response to the user input, the control circuitry 204 positions a graphical representation of the radially segmented electrodes (such as the graphical representation 104 depicted in **Fig. 6****)** over the map of the brain structure to create a graphical depiction of a desired depth and a desired radial orientation of the lead 12 within the brain tissue. The graphical depiction of the desired depth and the desired radial orientation may be substantially similar to that shown in **Fig. 7****,** and may be displayed by the user interface 202.

As briefly discussed above, the control circuitry may 204 be configured for dragging and dropping the graphical representation 104 of the set of radially segmented electrodes over the map 102 of the brain structure. For example, the control circuitry 204 may be configured for selecting the graphical representation 104 of the set of radially segmented electrodes by coupling a pointing device (e.g., a mouse cursor, finger, stylus, or the like) to the graphical representation 104 of the set of radially segmented electrodes. The graphical representation 104 of the set of radially segmented electrodes may then be dragged by moving the pointing device. The control circuitry 204 may further be configured for dropping the graphical representation 104 of the set of radially segmented electrodes by decoupling the pointing device from the dragged graphical representation 104 of the set of radially segmented electrodes.

If the method is not performed electronically, the map 102 may be drawn on paper, and the graphical representation 104 of the electrodes 26 may be positioned over the map 102 using stickers to represent the electrodes 26. Alternatively, the electrodes 26 may be drawn over the map 102 of the brain structure, or the map 102 and/or the graphical representation 104 of the electrodes 26 may be drawn on a transparent or translucent substrate that can be used as an overlay.

After the map 102 is generated and the graphical representation 104 of the electrodes 26 is positioned over the map 102, the lead 12 may be implanted using the map 102 with the electrode overlay for guidance. Thus, the lead 12 is implanted in the brain tissue in accordance with the desired depth and desired radial orientation.

In order to implant the lead 12 in the desired orientation, a device (e.g., a radially directional device) for determining the actual orientation of the electrodes 26 may be used. One of ordinary skill in the art may envision several different methods and devices for determining the actual orientation of the electrodes 26. Some exemplary embodiments of such methods and devices are discussed below.

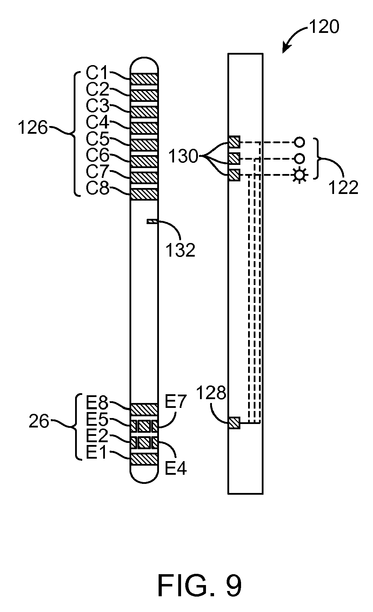

In one embodiment, shown in **Fig. 9****,** the radially directional device is a radially directional ruler 120 that uses electrical continuity in order to determine the orientation of the electrodes 26, as discussed in greater detail below. Each of the electrodes 26 on the distal end of the lead 12 is coupled to a different proximal ring contact 126. Lead and electrode configurations are standardized so that the distance between each electrode 26 and its associated ring contact 126 is known and fixed. Using this known distance, the radially directional ruler 120 comprises a single distal contact 128 and three proximal contacts 130. Each of the proximal contacts 130 is electrically coupled to the distal contact 128, as indicated by the dashed line. As such, the radially directional ruler 120 includes three open circuits represented by the dashed lines. The distances between the three proximal contacts 130 and the distal contact 128 on the radially directional ruler 120 correspond with, for example, the distances between contacts C5, C6, and C7 on the proximal end of the lead 12 and electrodes E5, E6, and E7 on the distal end of the lead 12. The lead 12 is positioned in contact with the radially directional ruler 120 such that one of the electrodes E5, E6, and E7 is electrically coupled to the distal contact 128 on the radially directional ruler 120, and the three contacts C5, C6, and C7 are each in contact with one of the proximal contacts 130 on the radially directional ruler 120. When such contact between the radially directional ruler 120 and the lead 12 is established, one of the open circuits on the radially directional ruler 120 is closed. In the example shown in **Fig. 9****,** the circuit between the distal contact 128 and the distal-most proximal contact 130 is closed by coupling with electrode E7 and contact C7 on the lead 12. An indicator, such as an LED 122 indicates which of the contacts C5, C6, or C7 is part of the closed circuit. In the example shown in **Fig. 9****,** the indicator 122 associated with contact C7 is activated when the circuit is closed. Because the user knows which electrode 26 is coupled with each of the contacts 126, the LED 122 indicates to the user that electrode E7 is the electrode that is in contact with the ruler 120. Thus, the actual orientation of the lead 12 is determined by using the radially directional ruler 120.

Although the exemplary radially directional ruler 120 depicted in **Fig. 9** is configured for use with a lead having a 1-3-3-1 electrode configuration, it should be well understood that the radially directional ruler could alternatively be configured for use with leads having other electrode configurations. For example, the sets of radially segmented electrodes may include at least two electrodes, and the radially directional ruler may include at least two proximal contacts.

After determining the actual orientation of the lead 12 using the radially directional ruler 120, the lead 12 may be inserted into the brain tissue through a guide cannula in the known radial orientation. For example, the actual radial orientation of the lead 12 may be indicated by placing a mark 132 on the proximal end of the lead 12, wherein the mark 132 is axially aligned with a known electrode. In the example shown in **Fig. 9****,** the mark 132 on the lead 12 indicates that electrode E7 is in axial alignment with the mark 132.

Alternatively, the radially directional ruler 120 with the lead 12 coupled thereto may be attached to a conventional stereotactic frame with the lead 12 in the known radial orientation. The known radial orientation of the lead 12 is then maintained while the lead 12 is inserted into the guide cannula. For example, the radially directional ruler 120 may include a longitudinal groove along its entire length. The size and shape of the groove may be similar to the size and shape of the lead 12 so that the lead 12 may fit snugly within the groove. The lead 12 is thus prevented from rotating during axial movement relative to the radially directional ruler 120.

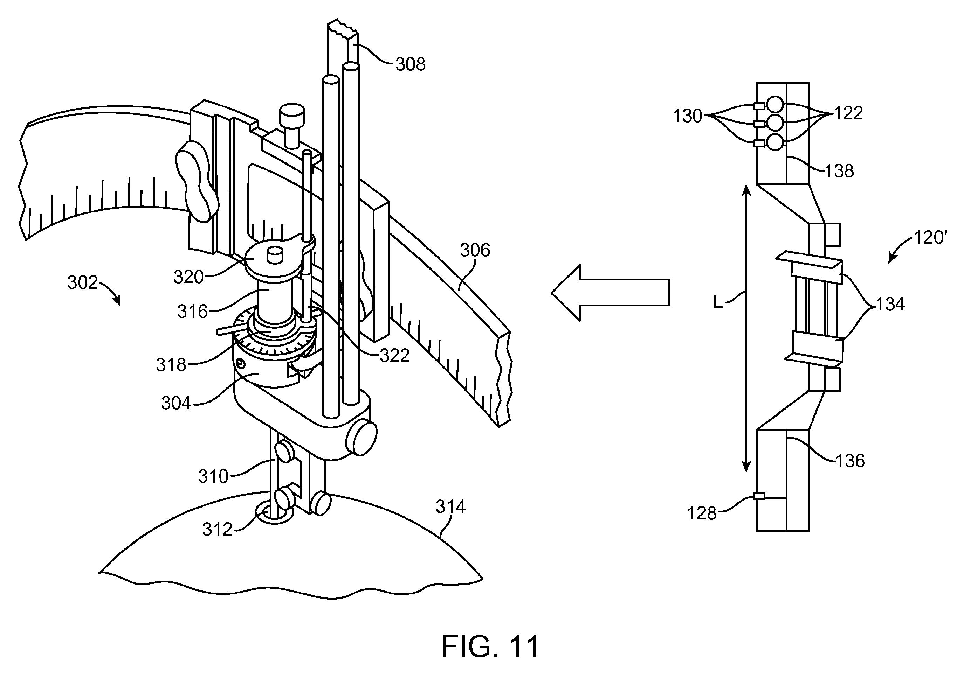

For example, and with reference to **Fig. 11****,** a radially directional ruler 120' is similar to the radially directional ruler 120 illustrated in **Fig. 9****,** with the exception that it is specifically designed to mount via a pair of brackets 134 to a stereotactic frame 300 on which a DBS lead insertion system 302 is mounted. The lead insertion system 302 includes a main base 304 coupled to a stereotactic frame 306 using a mounting structure 308. As shown, a guide cannula 310 is inserted through a burr hole 312 formed through the skull 314 of a patient thereby allowing passage of the lead 12 into the brain. The radially directional ruler 130 includes a lower groove 136 for accommodating the guide cannula 310, and an upper groove 138 for accommodating the lead 12.

The lead insertion system 302 further comprise a depth adjustment mechanism 316 for adjusting the depth of the lead 12 within the brain, and a longitude adjustment plate 318 for adjusting the longitudinal position of the lead 12 within the brain. The lead insertion system 302 further comprises a guiding plate 320 and a retaining rail 322 may also be provided to maintain a precise longitudinal direction of the lead 12. Further details describing the lead insertion system 302 are disclosed in U.S. Patent No. 7,369,899.

One exemplary method of affixing the lead 12 within the brain of the patient using the radially directional ruler 120' will now be described. First, the radially directional ruler 120' is mounted to the arc of the stereotactic frame 300 via the brackets 134. Then, the guide cannula 310 is inserted through the depth adjustment mechanism 316 and advanced into the lower groove 136 of the radially directional ruler 120' to a depth L. Then, the lead 12 is inserted into the guide cannula 310 and the proximal end of the lead 12 is disposed in the upper groove 138 of the radially directional ruler 120'. Next, the lead 12 is advanced into the lower groove 136, beyond the length L, such that the lead 12 extends beyond the distal end of the guide cannula 310. The lead 12 is continued to be advanced until one of the electrodes 26 at the distal end of the lead 12 passes over the distal contact 128 on the radially directional ruler 120', and the proximal contact 126 corresponding to the electrode 26 passes over one of the proximal contacts 130 on the radially directional ruler 120'. This completes the circuit and lights up one of the LEDs 122. The specific LED 122 that is lit will indicate which of the electrodes 26 is oriented facing the distal contact 128. Lastly, the lead 12 is secured and the depth adjustment mechanism 316 is operated to advance the guide cannula 310 into the brain. At this point, the radially directional ruler 120' can be removed, and the DBS procedure proceeds in a conventional manner, with the orientation of the lead 12 known.

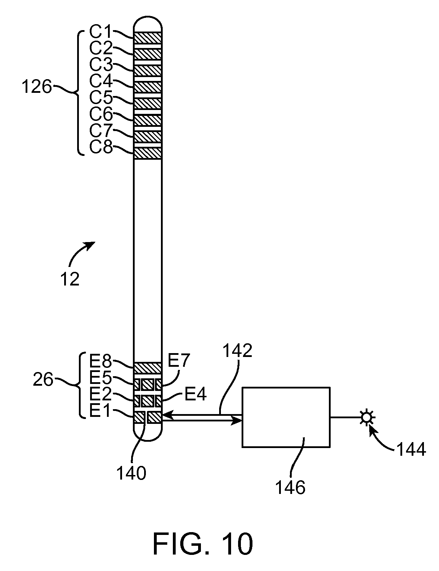

Rather than using electrical continuity, a radially directional device could use optical methods to determine the orientation of the electrodes 26, which however does not fall under the scope of the present invention. For example, such a radially directional device may include a laser. A laser marker on the lead 12, such as a small slit 140 formed in one of the ring electrodes E1, E8, as shown in **Fig. 10****,** may be detected by a laser 142 emitted by a laser emitter 146. The slit 140 formed in the ring electrode E1 or E8 may be aligned with one of the electrodes in the sets of radially segmented electrodes (e.g., E2 and E5, or E3 and E6, or E4 and E7). In the example shown in **Fig. 10****,** the slit 140 is formed in electrode E1 and is aligned with electrodes E3 and E6. The lead 12 is rotated relative to the laser 142 directed at the ring electrode E1 having the slit 140. An indicator 144, such as a LED or an audible alarm, may be used to indicate when the slit 140, and thus the electrodes aligned therewith, are in a known orientation (i.e., facing the laser 142). In this embodiment, the laser emitter 146 may be coupled to the stereotactic frame, and a stepper motor may be used to rotate the lead 12 until the indicator 144 is activated and the electrodes 26 are in a known radial orientation. Alternatively, similar to the method described above, the lead 12 may be marked when the electrodes 26 are in a known radial orientation.

In another embodiment (not shown) that uses optical methods to determine the orientation of the electrodes 26, the lead may incorporate optically opaque fenestrations, and the radially directional device may include an emitter-detector. As the lead 12 is rotated about its longitudinal axis, the fenestrations are counted by the emitter-detector in order to determine the orientation of the electrodes 26. The emitter-detector may be coupled to the stereotactic frame, and a stepper motor may be used to rotate the lead 12 about its longitudinal axis. Alternatively, similar to the method described above, the lead 12 may be marked when the electrodes 26 are in a known radial orientation.

In the above embodiments, the stepper motor may have very fine resolution to control the radial orientation of the lead 12. The control of the stepper motor can be manifested through a remote interface, permitting a user to control radial orientation without impinging on the sterile field of the operating theater. The stepper motor may be controlled automatically by a computer which uses the microelectrode recording data to help direct radial orientation.

Although particular embodiments of the present invention have been shown and described, it will be understood that it is not intended to limit the present invention to the preferred embodiments, and it will be obvious to those skilled in the art that various changes and modifications may be made without departing from the scope of the present invention. Thus, the present invention is intended to cover alternatives, modifications, and equivalents, which may be included within the scope of the present invention as defined by the claims.

## Claims

1. A device for determining a radial orientation of a lead (12), wherein the lead (12) comprises at least one set of radially segmented electrodes (E2; E3; E4; E5; E6; E7) on a distal end of the lead (12), and at least two contacts (130) on a proximal end of the lead (12), each electrode (E2; E3; E4; E5; E6; E7) in the set of radially segmented electrodes (E2; E3; E4; E5; E6; E7) being coupled to one of the contacts on the proximal end of the lead (12), **characterised in that** the device comprises:
a radially directional ruler (120; 120') comprising a distal contact and at least two proximal contacts; and
an indicator (122) for indicating which one of the electrodes in the set of radially segmented electrodes (E2; E3; E4; E5; E6; E7) is in contact with the radially directional ruler (120; 120'), wherein the radially directional ruler is configured for contacting the lead such that the ruler distal contact is coupled to one of the electrodes in the set of radially segmented electrodes, and the ruler proximal contacts are coupled to the lead proximal contacts, thereby forming a closed circuit between the one of the electrodes coupled to the ruler distal contact and the lead proximal contact that corresponds to the one of the electrodes, and wherein the indicator (122) is configured for indicating which one of the lead proximal contacts is in the closed circuit.

2. The device of claim 1, wherein the indicator (122) is an LED.

3. The device of any of claims 1-2, wherein the set of radially segmented electrodes (E2; E3; E4; E5; E6; E7) comprises three electrodes and the radially directional ruler comprises three proximal contacts.

4. The device of any of claims 1-3, wherein the radially directional ruler (120; 120') comprises at least two open circuits, and wherein each open circuit comprises the distal contact and one of the proximal contacts.

5. The device of any of claims 1-4, wherein the radially directional ruler (120; 120') is configured for being attached to a stereotactic frame.

## Patentansprüche

1. Vorrichtung zur Bestimmung einer radialen Ausrichtung einer Leitung (12), wobei die Leitung (12) mindestens einen Satz radial segmentierter Elektroden (E2; E3; E4; E5; E6; E7) an einem distalen Ende der Leitung (12) und mindestens zwei Kontakte (130) an einem proximalen Ende der Leitung (12) aufweist, wobei jede Elektrode (E2; E3; E4; E5; E6; E7) im Satz radial segmentierter Elektroden (E2; E3; E4; E5; E6; E7) mit einem der Kontakte am proximalen Ende der Leitung (12) gekoppelt ist, **dadurch gekennzeichnet, dass** die Vorrichtung aufweist:
radial gerichtetes Lineal (120; 120'), das einen distalen Kontakt und mindestens zwei proximale Kontakte aufweist; und
eine Anzeigevorrichtung (122) zum Anzeigen, welche der Elektroden im Satz radial segmentierter Elektroden (E2; E3; E4; E5; E6; E7) sich mit dem radial gerichteten Lineal (120; 120') in Kontakt befindet, wobei das radial gerichtete Lineal konfiguriert ist, mit der Leitung so in Kontakt zu kommen, dass der distale Linealkontakt mit einer der Elektroden im Satz radial segmentierter Elektroden gekoppelt ist, und die proximalen Linealkontakte mit den proximalen Leitungskontakten gekoppelt sind, wodurch zwischen der einen der Elektroden, die mit dem distalen Linealkontakt gekoppelt ist, und dem proximalen Leitungskontakt, der der einen der Elektroden entspricht, ein geschlossener Stromkreis gebildet wird, und wobei die Anzeigevorrichtung (122) konfiguriert ist anzuzeigen, welcher der proximalen Leitungskontakte sich im geschlossenen Stromkreis befindet.

2. Vorrichtung nach Anspruch 1, wobei die Anzeigevorrichtung (122) eine LED ist.

3. Vorrichtung nach einem der Ansprüche 1 bis 2, wobei der Satz radial segmentierter Elektroden (E2; E3; E4; E5; E6; E7) drei Elektroden aufweist und das radial gerichtete Lineal drei proximale Kontakte aufweist.

4. Vorrichtung nach einem der Ansprüche 1 bis 3, wobei das radial gerichtete Lineal (120; 120') mindestens zwei offene Stromkreise aufweist, und wobei jeder offene Stromkreis den distalen Kontakt und einen der proximalen Kontakte aufweist.

5. Vorrichtung nach einem der Ansprüche 1 bis 4, wobei das radial gerichtete Lineal (120; 120') konfiguriert ist, an einem stereotaktischen Rahmen angebracht zu werden.

## Revendications

1. Dispositif pour la détermination d'une orientation radiale d'une sonde (12), où ladite sonde (12) comprend au moins un ensemble d'électrodes (E2 ; E3 ; E4 ; E5 ; E6 ; E7) radialement segmentées à une extrémité distale de la sonde (12), et au moins deux contacts (130) à une extrémité proximale de la sonde (12), chaque électrode (E2 ; E3 ; E4 ; E5 ; E6 ; E7) de l'ensemble d'électrodes (E2 ; E3 ; E4 ; E5 ; E6 ; E7) radialement segmentées étant reliée à un des contacts à l'extrémité proximale de la sonde (12), **caractérisé en ce que** ledit dispositif comprend :
une règle radialement directionnelle (120 ; 120') comprenant un contact distal et au moins deux contacts proximaux ; et
un indicateur (122) destiné à indiquer laquelle des électrodes de l'ensemble d'électrodes (E2 ; E3 ; E4 ; E5 ; E6 ; E7) radialement segmentées est en contact avec la règle radialement directionnelle (120 ; 120'), ladite règle radialement directionnelle étant prévue pour contacter la sonde de manière à relier le contact distal de règle à une des électrodes de l'ensemble d'électrodes radialement segmentées, et à relier les contacts proximaux de règle aux contacts proximaux de sonde, en formant ainsi un circuit fermé entre l'électrode reliée au contact distal de règle et le contact proximal de sonde correspondant à ladite électrode, et l'indicateur (122) étant prévu pour indiquer lequel des contacts proximaux de sonde est dans le circuit fermé.

2. Dispositif selon la revendication 1, où l'indicateur (122) est une LED.

3. Dispositif selon la revendication 1 ou la revendication 2, où l'ensemble d'électrodes (E2 ; E3 ; E4 ; E5 ; E6 ; E7) radialement segmentées comprend trois électrodes et où la règle radialement directionnelle comprend trois contacts proximaux.

4. Dispositif selon l'une des revendications 1 à 3, où la règle radialement directionnelle (120 ; 120') comprend au moins deux circuits ouverts, et où chaque circuit ouvert comprend le contact distal et un des contacts proximaux.

5. Dispositif selon l'une des revendications 1 à 4, où la règle radialement directionnelle (120 ; 120') est prévue pour être fixée à un cadre stéréotaxique.
